# EUROPEAN PATENT APPLICATION

(11) **EP 3 401 673 A1**
(43) Date of publication of application: **14.11.2018**
(21) Application number: 17170143.6
(22) Date of filing: 09.05.2017
(51) Int. Cl.: G01N 27/02, G01N 33/12

(54) **DEVICE AND METHOD FOR DETERMINING MEAT FRESHNESS**

(71) Applicant: Koninklijke Philips N.V., 5656 AE Eindhoven (NL)
(72) Inventor: FENG, Haitao, 5656 AE Eindhoven (NL); LUO, ZhongChi, 5656 AE Eindhoven (NL)
(74) Representative: de Haan, Poul Erik

(57) **Abstract**

The present invention relates to a device and method for determining meat freshness in a convenient, fast-response and accurate manner. The device comprises an AC generator (2) for generating an AC signal, two electrodes (4, 5) for placement into a piece of meat to provide the AC signal into the piece of meat, a measurement circuit (6) for measuring voltage between the electrodes and current through the electrodes, an impedance calculation circuit (11) for calculating the electrical impedance of the piece of meat from the measured voltage and current, a TVBN estimation circuit (13) for estimating the total volatile basic nitrogen, TVBN, value of the piece of meat from the calculated impedance based on a predetermined transfer function, and a freshness determination circuit (16) for determining freshness of the piece of meat from the estimated TVBN value.

## Description

### FIELD OF THE INVENTION

The present invention relates to a device and method for determining meat freshness.

### BACKGROUND OF THE INVENTION

Meat freshness sensing is a common unmet consumer need globally. On the one hand, a lot of people get sick due to the consumption of spoiled meat. On the other hand, a lot of meat that is still edible is simply wasted just because it exceeds expiration date or the consumer is not sure if it is still edible or not. If food freshness can be evaluated in a simple way, it will generate great benefit for both of people's health and reduction of food waste.

It is generally difficult to judge meat freshness status simply from sensory indexes, such as color, flavor and texture, since obvious changes of these properties will occur only when the meat is already spoiled. Although total volatile basic nitrogen (TVBN) is the direct indicator of meat freshness, it can only be measured by a professional operator in a laboratory and measurement needs a relatively long time, which makes such direct measurement unsuitable for onsite test or consumer use. There are the same issues in the determination of other meat freshness indicators such as total viable bacteria concentration (TVC) and biogenic amine. Overall, there is no suitable testing method to objectively determine meat freshness quickly that can be used by ordinary people.

### SUMMARY OF THE INVENTION

It is an object of the present invention to provide a device and method that enable ordinary people to determine meat freshness in a convenient, fast-response and accurate manner.
In a first aspect of the present invention a device for determining meat freshness is presented comprising:
- an AC generator for generating an AC signal,
- two electrodes for placement into a piece of meat to provide the AC signal into the piece of meat,
- a measurement circuit for measuring voltage between the electrodes and current through the electrodes,
- an impedance calculation circuit for calculating the electrical impedance of the piece of meat from the measured voltage and current,
- a TVBN estimation circuit for estimating the total volatile basic nitrogen, TVBN, value of the piece of meat from the calculated impedance based on a predetermined transfer function, and
- a freshness determination circuit for determining freshness of the piece of meat from the estimated TVBN value.
In a further aspect of the present invention a corresponding method for determining meat freshness is presented.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, computer program and medium have similar and/or identical preferred embodiments as the claimed device, in particular as defined in the dependent claims and as disclosed herein.

The present invention is based on the idea to exploit the finding that impedance is related to meat quality including freshness indicator TVBN to rather accurately predict the freshness of an unknown sample of meat. With the disclosed solution, a predetermined transfer function, which may be a quite simple equation, is used, said transfer function defining the relationship between impedance and TVBN, which covers both of fresh and spoiled sample. Thus, the impedance is of a piece of meat is determined by measurement of voltage and current, while an AC signal (current or voltage signal at one or more frequencies) is provided to the piece of meat. From the impedance, the TVBN can be estimated, which then indicates freshness of the piece of meat. The effectiveness of this approach has been verified with unknown samples.

Generally, a single pin per electrode is sufficient. An ideal electrode may be configured as a piece of a sufficiently large metallic plate, equivalent to infinite parallel pins. According to a more cost effective and user friendly embodiment each of said two electrodes comprises two or more or more pins galvanically connected. Two or more pins contribute to current homogeneity and thus improve measurement accuracy. For instance, three pins per electrode may be a good compromise, which is convenient to use with good measurement performance.

In an embodiment said AC generator is configured to generate an AC signal in the range of 100 Hz to 100 kHz, preferably in the range of 200 Hz to 10 kHz. These frequency ranges have been shown to provide reasonable results in the detection of freshness.

In a preferred embodiment said AC generator is configured to generate two or more AC signals at different frequencies for providing to the piece of meat via the two electrodes, wherein said measurement circuit to measure two or more voltage and current values when said two or more AC signals are provided to the piece of meat, wherein said impedance calculation circuit is configured to calculate two or more electrical impedance values from the two or more measured voltage and current values, wherein said TVBN estimation circuit is configured to estimate two or more TVBN values from the two or more calculated impedance values, and wherein said freshness determination circuit is configured to determine two or more freshness values two or more estimated TVBN values and for determining a final freshness value from said two or more determined freshness values. The use of two or more AC signals at different frequencies increases the accuracy of the freshness determination.

In one embodiment using two or more AC signals, said freshness determination circuit is configured to determine the final freshness value by selecting one of said two or more determined freshness values or by combining, e.g. averaging, said two or more determined freshness values.

In another embodiment using two or more AC signals, said TVBN estimation circuit is configured to use an individual transfer function assigned to a particular frequency for estimating the TVBN value from an impedance value calculated from voltage and current measured while an AC signal at said particular frequency is provided to the piece of meat. There may, for instance, be a number of transfer functions for different frequencies. Depending on the frequency (or frequencies) used by the AC signal generator, the corresponding transfer function(s) will then be used for the evaluation.

The AC generator may further be configured to generate said two or more AC signals at different frequencies simultaneously or (preferably) subsequently.

In an embodiment said TVBN estimation circuit is configured to use a first order function as transfer function. This makes determination of the TVBN value efficient and fast, i.e. can be easily implemented without much computation efforts, but nevertheless provides good results.

The device may further comprise a meat type input configured to receive a meat type indicator indicating the type of meat. For instance, a user may provide such an indicator via a user interface, e.g. touch screen, buttons, etc. Alternatively, the type of meat may be detected automatically, e.g. optically via a camera or via a detection of the impedance range, which is different for different types of meat.

Accordingly, said AC generator may be configured to set the amplitude and/or frequency of the AC signal based on the type of meat indicated by meat type indicator and/or said TVBN estimation circuit may be configured to select a transfer function from among a plurality of transfer functions for different types of meat base on the type of meat indicated by meat type indicator. This further improves the accuracy of the detection of freshness.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a first embodiment of a device according to the present invention,
Fig. 2 shows a schematic diagram of a second embodiment of a device according to the present invention,
Fig. 3 shows a flow chart of a method for determining the transfer function,
Fig. 4 shows a graph of the coefficient of correlation analysis for different frequencies, and
Fig. 5 shows a graph of the correlation between TVBN and impedance at 5 kHz.

### DETAILED DESCRIPTION OF THE EMBODIMENTS

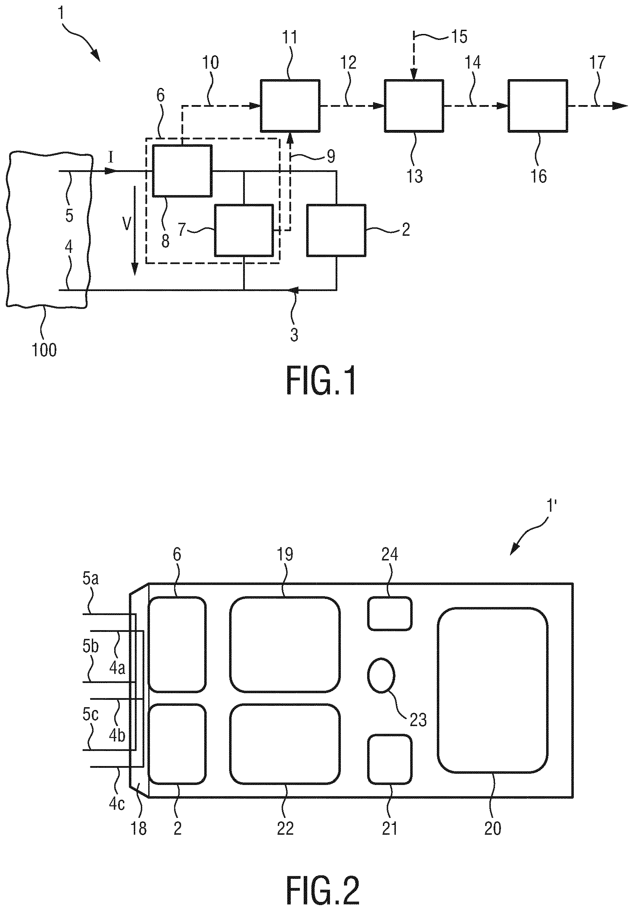

Fig. 1 shows a schematic diagram of a first embodiment of a device 1 according to the present invention. The device 1 comprises an AC generator 2, e.g. a current source or a voltage source, for generating an AC signal 3. Two electrodes 4, 5 (each having one pin in this embodiment) are provided for placement into a piece of meat 100 to provide the AC signal 3 (a current or voltage signal) into the piece of meat 100. The device 1 further comprises a measurement circuit 6 for measuring voltage V between the electrodes 4, 5 and current I through the electrodes 4, 5. The measurement circuit 6 may e.g. comprise a voltmeter 7 and an amperemeter 8. From voltage information 9 indicating the measured voltage V and current information 10 indicating the measured current I an impedance calculation circuit 11 calculates the electrical impedance 12 of the piece of meat 100. A TVBN estimation circuit 13 is provided for estimating the total volatile basic nitrogen (TVBN) value 14 of the piece of meat 100 from the calculated impedance 12 based on a predetermined transfer function 15, which may be stored in a storage medium (not shown). Finally, a freshness determination circuit 16 determines freshness 17 of the piece of meat 100 from the estimated TVBN value 14.

The AC generator 2 generates an AC signal preferably having one or more frequencies (simultaneously or subsequently) in the frequency range 100-100 kHz, preferably in the frequency range 200-10 kHz. Two electrodes 4, 5 are inserted into the meat 100. Thus, AC generator 2, electrodes 4, 5 and meat 100 form a closed circuit. The voltage V between the electrodes 4, 5 and the current I in the circuit together with their phases can be measured simultaneously, and the impedance 12 of the meat 100 can be calculated accordingly.

The impedance calculation circuit 11, the TVBN estimation circuit 13 and the freshness determination circuit 16 may be implemented in hard- and/or software, e.g. by separate processors or by a common processor or computer.

Fig. 2 shows a schematic diagram of a second embodiment of a device 1' according to the present invention. In this embodiment each of the electrodes 4, 5 has three pins 4a, 4b, 4c and 5a, 5b, 5c, respectively, the three pins of an electrode being galvanically connected to on the same potential. The use of three (or at least two) pins per electrode increases the accuracy of the measurement. An insulating plate 18 is provided to insulate the electrodes 4 and 5 from each other.

A processing unit 19 programmed with a loaded program implements the impedance calculation circuit 11, the TVBN estimation circuit 13 and the freshness determination circuit 16. The processing unit 19 can set the AC generator 2 to predefined frequencies. Further, the processing unit 19 stores the digital measurement results and interprets the measurement results with built-in algorithm to express as meat freshness level 17. The processing unit 19 sends the final results to a display 20 or to an external device (not shown), such as a mobile phone or server, using a transmitter 21 through wireless communication, such as Bluetooth or WiFi. A battery 22 provides electric energy and a switch 23 enable switching on and off the device 1'.

Optionally, a meat type input 24, e.g. a user interface, is provided for receiving a meat type indicator indicating the type of meat. The AC generator 2 then sets the amplitude and/or frequency of the AC signal based on the type of meat indicated by meat type indicator and/or the TVBN estimation circuit selects a transfer function from among a plurality of transfer functions for different types of meat base on the type of meat indicated by meat type indicator.

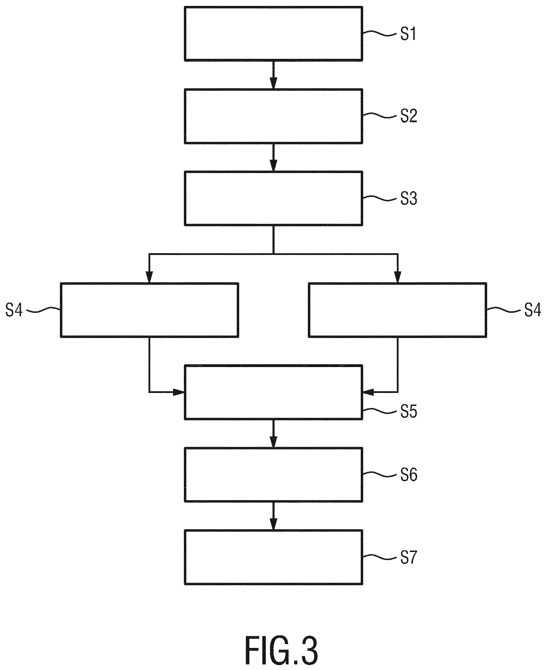

Fig. 3 shows a flow chart of a method for determining the transfer function, i.e. for creating an algorithm to transform impedance results to meat freshness level.

In a first step S1, fresh meat samples are prepared and stored at a low temperature, e.g. at 4°C, for several days, e.g. up to 10 days. In a second step S2, impedance and TVBN are measured for each sample at several days, e.g. at day 1, 3, 5, 7 and 10. Samples of each day constitute a subgroup. For each subgroup several samples, e.g. at least ten samples, are needed. In a third step S3a principal component analysis (PCA) is made to analyse the coefficient of correlation for different frequencies. Frequencies with high coefficient are chosen for the following analysis. In a fourth step S4, both of the impedance and TVBN values within one subgroup are averaged. In a fifth step S5, a correlation is set up with the average values of impedance and TVBN and a transfer function is obtained.

The transfer function can be used to predict TVBN value based on impedance information in a sixth step S6. A meat sample is defined as fresh in a seventh step S7 when the TVBN is lower than a threshold, e.g. lower than 15 mg per 100g of meat. It is defined as spoiled when TVBN is higher than the threshold, e.g. higher than 15 mg per 100g of meat.

The prediction accuracy of the transfer function has been verified with independent unknown samples. For the real application suitable frequencies may be chosen. It may be a single frequency or a combination of different frequencies.

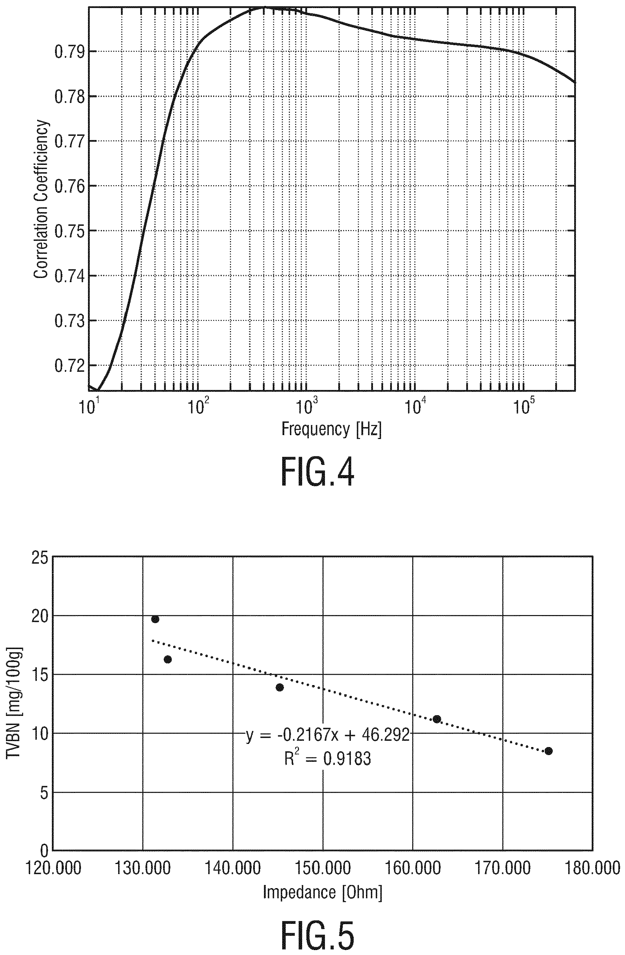

To obtain experiment results, first the PCA is analysed to determine a more suitable frequency. It is found that 100 Hz -100 kHz showed higher correlation. More specifically, the frequency of 200 Hz - 10 kHz is better. The coefficient of the correlation analysis for different frequencies is shown in Fig. 4. Then, the correlation between TVBN and impedance is analysed for frequencies of 200, 1000, 5000, 100000 Hz. Fig. 5 shows the results of 5000 Hz as an example.

PCA (principle component analysis) and PLS (partial linear square) are alternatives to build the (linear) relationship/model between impedance and freshness. These algorithms extract frequencies at which the impedances are most relevant to freshness, and thus build a linear map from impedances at selected frequencies to freshness evaluated by TVBN. In this step, the frequency selection is done in a quantitative way so there is no need for end users to do it.

After the transfer functions at different frequencies are obtained, they may be verified with independent unknown samples that covered different freshness level. The following table is the summary of exemplary results. From the table, it can be found that 1 kHz and 5 kHz obtained good results. The total accuracy reached over 89%. Prediction accuracy for spoiled sample also reached over 97%. The total accuracy is hereby indicated by R², which is a coefficient of determination often used in statistics to evaluate how close the estimated data are to the fitted regression line or curve. As a rule of thumb, the closer to 1 the R² is the better the fitting result.

| | | Accuracy for fresh sample | Accuracy for spoiled sample | Total Accuracy |
|---|---|---|---|---|
| | | (n=34) | (n=31) | (n=65) |
| 200 Hz | y = -0.1457x + 45.282 R² = 0.9076 | 64.7% | 100.0% | 81.5% |
| 1 KHz | y = -0.2017x + 46.833 R² = 0.9171 | 82.4% | 97.0% | 89.2% |
| 5 KHz | y = -0.2167x + 46.292 R² = 0.9183 | 82.4% | 97.0% | 89.2% |
| 100 KHz | y = -0.2252x + 46.454 R² = 0.9194 | 67.6% | 100.0% | 83.1% |

The transfer function may be a simple function, such as a first order equation of the form y = a x + b, where the values of the parameters a and b are found through the above described (see Fig. 3) method for determining the transfer function. For different types of meat and/or for different frequencies (of the AC signal) different values of the parameter a and b maybe found and then used in the device for determining the meat freshness.

The present invention enables a convenient, fast-response and accurate evaluation of meat freshness in time and avoiding food waste.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for determining meat freshness comprising:
- an AC generator (2) for generating an AC signal,
- two electrodes (4, 5) for placement into a piece of meat to provide the AC signal into the piece of meat,
- a measurement circuit (6) for measuring voltage between the electrodes and current through the electrodes,
- an impedance calculation circuit (11) for calculating the electrical impedance of the piece of meat from the measured voltage and current,
- a TVBN estimation circuit (13) for estimating the total volatile basic nitrogen, TVBN, value of the piece of meat from the calculated impedance based on a predetermined transfer function, and
- a freshness determination circuit (16) for determining freshness of the piece of meat from the estimated TVBN value.

2. Device as claimed in claim 1,
wherein each of said two electrodes (4, 5) comprises two or more pins galvanically connected.

3. Device as claimed in claim 1 or 2,
wherein said AC generator (2) is configured to generate an AC signal in the range of 100 Hz to 100 kHz.

4. Device as claimed in any one of the preceding claims,
wherein said AC generator (2) is configured to generate an AC signal in the range of 200 Hz to 10 kHz.

5. Device as claimed in any one of the preceding claims,
wherein said AC generator (2) is configured to generate two or more AC signals at different frequencies for providing to the piece of meat via the two electrodes, wherein said measurement circuit (6) is configured to measure two or more voltage and current values when said two or more AC signals are provided to the piece of meat,
wherein said impedance calculation circuit (11) is configured to calculate two or more electrical impedance values from the two or more measured voltage and current values,
wherein said TVBN estimation circuit (13) is configured to estimate two or more TVBN values from the two or more calculated impedance values, and
wherein said freshness determination circuit (16) is configured to determine two or more freshness values two or more estimated TVBN values and for determining a final freshness value from said two or more determined freshness values.

6. Device as claimed in claim 5,
wherein said freshness determination circuit (16) is configured to determine the final freshness value by selecting one of said two or more determined freshness values or by combining, e.g. averaging, said two or more determined freshness values.

7. Device as claimed in claim 5 or 6,
wherein said TVBN estimation circuit (13) is configured to use an individual transfer function assigned to a particular frequency for estimating the TVBN value from an impedance value calculated from voltage and current measured while an AC signal at said particular frequency is provided to the piece of meat.

8. Device as claimed in any one of claims 5 to 7,
wherein said AC generator (2) is configured to generate said two or more AC signals at different frequencies simultaneously or subsequently.

9. Device as claimed in any one of the preceding claims,
wherein said TVBN estimation circuit (13) is configured to use a first order function as transfer function.

10. Device as claimed in any one of the preceding claims,
further comprising a meat type input (22) configured to receive a meat type indicator indicating the type of meat.

11. Device as claimed in claim 10,
wherein said AC generator (2) is configured to set the amplitude and/or frequency of the AC signal based on the type of meat indicated by the meat type indicator.

12. Device as claimed in claim 10 or 11,
wherein said TVBN estimation circuit (13) is configured to select a transfer function from among a plurality of transfer functions for different types of meat base on the type of meat indicated by the meat type indicator.

13. Method for determining meat freshness comprising:
- generating an AC signal,
- providing the AC signal into a piece of meat by two electrodes for placement into the piece of meat,
- measuring voltage between the electrodes and current through the electrodes,
- calculating the electrical impedance of the piece of meat from the measured voltage and current,
- estimating the TVBN value of the piece of meat from the calculated impedance based on a predetermined transfer function, and
- determining freshness of the piece of meat from the estimated TVBN value.

14. Computer program comprising program code means for causing a computer to carry out the steps of the method as claimed in claim 13 when said computer program is carried out on the computer.
